(19)

Europäisches
Patentamt
European
Patent Office
Office européen
des brevets

(11) **EP 2 100 624 B1**

(12) **EUROPEAN PATENT SPECIFICATION**

(45) Date of publication and mention
of the grant of the patent:
**09.10.2019   Bulletin 2019/41**

(51) Int Cl.:
*A61L 9/20* *(2006.01)*     *A61L 9/22* *(2006.01)*

(21) Application number: **09397506.8**

(22) Date of filing: **12.03.2009**

(54) **Device and method for disinfecting air**

Gerät und Verfahren zum Desinfizieren von Luft

Dispositif et méthode pour désinfecter l'air

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **14.03.2008   FI 20085224**

(43) Date of publication of application:
**16.09.2009   Bulletin 2009/38**

(73) Proprietor: **ProAir Oy Ltd
10210 Inkoo (FI)**

(72) Inventor: **Rousi, Jussi
33100, Tampere (FI)**

(74) Representative: **Berggren Oy, Tampere et al
Visiokatu 1
33720 Tampere (FI)**

(56) References cited:
**US-A1- 2004 134 354     US-A1- 2005 186 108
US-A1- 2007 041 882     US-A1- 2007 253 860
US-A1- 2007 266 855**

## Description

<u>Field of the invention</u>

The invention relates to the disinfection of air, particularly dry disinfection. More precisely, the invention relates to a dry disinfection device and a method for the disinfection of air.

<u>Background of the invention</u>

A variety of methods can be used for the decontamination of air, including for example UV and filtering methods. Ozone or negative ions can be also used for the decontamination of air.

In practice, ionization refers to the production of negative ions in the air. In nature, these ions are produced e.g. by cosmic radiation, radioactive radiation from the ground, UV light, charging caused by wind friction, electric discharges, combustion, and strong electric fields. In clean air, the lifetime of a negative ion is normally 100 to 1000 seconds. Negative ions are decomposed e.g. by such combustion processes in which particles are formed. For example, the smoking of one cigarette may reduce the ion concentration of a room to a level lower than one per mille of the starting level.

In the decontamination of air based on ionization, reactive oxygen species are supplied into the air to destroy various microorganisms and odorous organic compounds by oxidation. The ionization produces such reactive oxygen species which are not harmful to the human body. Consequently, ionization does not involve such concentration limits as ozonization. Another advantage of ionization is the negative charging of particles in the air. Thus, the particles accumulate and adhere to surfaces, escaping from the air.

Ozone ($O_3$), in turn, is a triatomic form of oxygen with a strongly oxidizing property. In nature, ozone is formed e.g. by the effect of solar UV radiation in the upper atmosphere and, on the earth, for example in connection with lightning strokes. Ozone oxidizes several odorous compounds to an odourless form, and ozone is thus a good deodorizer. Furthermore, even low ozone contents have strong antiseptic properties. Even in small concentrations, ozone is very toxic to all viruses, anaerobic bacteria and fungi. Even low concentrations of only 0.05 ppm may have antiseptic properties in long term if the ozone can be evenly distributed.

For people, long-term inhalation of large ozone contents causes damage *e.g.* in lung tissues, and therefore the ozone concentration must be limited. The allowed range for the ozone concentration varies generally from 0.05 ppm to 0.1 ppm. When high ozone concentrations (1 to 5 ppm) are used, one can stay in such a room only temporarily. However, ozone is a reactive compound that is degraded relatively fast, so that the concentration of 1 ppm will drop to the allowed range in only a few hours, depending on the conditions. Therefore, efficient ozonization that is sufficient for sterilization can be performed, for example, after a working day, in which case the room will be suitable for working on the next day. Another alternative is to decompose the ozone catalytically. By means of an atomizer, even an ozone concentration of 7 ppm will drop to the safe range in about 20 minutes. With appropriate equipment, such ozone decontamination can thus be performed in even the most urgent cases, or in places where e.g. operating theatres or other rooms with hygienic requirements are only vacant for short times.

Various devices have been developed for ozonization. Typically, the ozone is produced by UV light and distributed in the room by a fan.

US20070041882 discloses an onsite chemistry air filtration system comprising a photochemical filtration section, where UV lamps generate bio-destruction and surface photochemical activity on a photocatalytic material and provide a radiation source to irradiate airborne molecules passing through the section and to energize their states to promote reactions. In US20070041882 there are UV lamps and/or other ozone and radical generation devices that are used to generate hydroxyl radicals among others. The photochemical filtration section also may contain electrostatic plates configured to capture particulate matter.

<u>Brief summary of the invention</u>

It is an aim of the solution according to the invention to present a new air disinfection solution that is easy to install.

It is an aim of the present invention to provide a system that is easily integrated in a ventilation system and that is also efficient.

To achieve this aim, the dry disinfection device according to the invention is primarily characterized in what will be presented in the independent claim 1. The method according to the invention is, in turn, primarily characterized in what will be presented in the independent claim 10. The other, dependent claims will present some preferred embodiments of the invention.

The invention is based on the idea that air is disinfected by UV radiation, ozonization, ionization, and hydroxyl radicals (OH radicals) simultaneously. The basic idea of the invention is to provide a dry disinfection device whose operation is based on UV radiation, ozonization, ionization and hydroxyl radicals (OH radicals).

The dry disinfection device according to the invention comprises at least a process chamber, wherein the process chamber is part of a ventilation duct and wherein the air to be decontaminated is guided through the process chamber and wherein the process chamber comprises at least a UV radiator arranged to emit at a first wavelength and arranged to produce ozone, the process chamber further comprising a UV radiator arranged to emit at a second wavelength, which second wavelength is different from the first wavelength; an ionizer arranged to generate ions for the process chamber, wherein the process chamber comprises at least dimensions for a length and a cross-sectional area, and wherein air flowing the process chamber has a retention time, and a diffusion collector arranged to remove particles from the air. The process chamber is dimensioned by the following dimensioning rules based on the measurements of the device and the duct and a retention time. The equation is

$$\text{Retention time} = \frac{L * A}{V},$$

wherein, retention time stands for a time of air flowing in the process chamber, and wherein the retention time is 0.4 - 2 seconds, L stands for the length of the process chamber, A stands for the cross-sectional area in the process chamber per the cross-sectional area in the duct, and V stands for a rate of the air flowing through the duct, and wherein $A \geq 1$ and $0 \leq V \leq 10$ m/s.

In an embodiment, the first wavelength is shorter than 200 nm, preferably 180 to 190 nm. In an embodiment, the wavelength of 185 nm is used for ozone production.

In an embodiment, the second wavelength is longer than 200 nm, preferably 245 to 260 nm. In an embodiment, *inter alia,* the wavelength of 253.7 nm is used for killing bacteria.

In the solution according to the invention, a large quantity of negative ions is generated into the air. Upon an impact of oxygen atoms and the negative ions, superoxide radicals are formed. These superoxide radicals react with aqueous vapour in the air, forming perhydroxyl and hydroxyl radicals. Also according to the invention, ozone is generated into the same air. The production of hydroxyl radicals is accelerated further when the superoxide radicals react with ozone. The production of ozone and negative ions takes place closely in the same room. Thus, the different reactions of ozone and the negative ions take a time that is as long as possible. The device according to the invention releases hydroxyl radicals into its environment, as well as advantageously also negative ions and ozone. The radicals oxidize organic molecules strongly, thereby decontaminating the air. The UV radiation, the negative ions and the ozone also decontaminate the air for their part. The forming hydroxyl radicals are among the most antiseptic compounds. For example in a deodorizing process, ozone and the radicals accumulate and decompose the organic compounds they detect, including e.g. odours. In the oxidizing reaction, the odorous substance turns to harmless carbon dioxide, aqueous vapour and oxygen.

In an embodiment, the process chamber further comprises a layer that forms the inner wall of the process chamber and is arranged to reflect the radiation of UV radiators back to an air flow. In an embodiment, the layer is also arranged to function as a counter electrode for a discharge tip used as an ionizer.

In an embodiment, the device also comprises a power supply arranged to supply electricity to the first UV radiator, the second UV radiator and the discharge tip.

In an embodiment, the device also comprises a frame structure to which the first UV radiator, the second UV radiator and the discharge tip are fixed. In an embodiment, the frame structure is connected to a cover structure that is used at the process chamber as a lid for the ventilation duct and as a mounting support for the device.

The different embodiments of the above-described arrangement, taken separately and in various combinations, provide several advantages. In one embodiment, a significant advantage lies in the fact that it can be easily installed as a part of the ventilation system of a building, even in existing systems.

The dry disinfection solution according to the invention makes very efficient decontamination of air possible even in rooms with people. Furthermore, the dry disinfection solution according to the invention provides many other advantages, including for example:

- the decomposition and elimination of harmful particles and volatile organic compounds, and their conversion to a harmless form,
- the elimination of odours,
- the inactivation of microbes,
- low energy costs,
- minimized accumulation of particles onto surfaces,
- no production of harmful reactants or side products.

Description of the drawings

In the following, the invention will be described in more detail with reference to the appended basic drawings, in which

Fig. 1                        shows the main components of the structure of a dry disinfection device,

Fig. 2                        shows the placement of the arrangement of Fig. 1 in a ventilation duct,

Figs. 3a, 3b and 3c    show an advantageous embodiment of the dry disinfection device,

Figs. 4a, 4b and 4c    show another advantageous embodiment of the dry disinfection device,

Fig. 5                        shows a third embodiment of the dry disinfection device,

Fig. 6                        shows a fourth embodiment of the dry disinfection device.

For the sake of clarity, the drawings only show the details necessary for understanding the invention. The structures and details that are not necessary for understanding the invention but are obvious for anyone skilled in the art have been omitted in the figures in order to emphasize the characteristics of the invention.

Detailed description of the invention

Ultraviolet radiation, or more briefly UV radiation, is electromagnetic radiation. The wavelength of UV radiation (100 to 380 nm) is shorter than that of visible light but longer than that of x-rays. The short wavelength means that the frequency of UV radiation is high. UV radiation is divided into three ranges of radiation, primarily according to its effects on human health and the environment, i.e. its biological effects: UVA radiation (wavelength 315 to 380 nm), UVB radiation (wavelength 280 to 315 nm) and UVC radiation (wavelength 100 to 280 nm).

In this description, the term UV radiator refers to a device for emitting UV radiation. For example, a UV radiator may be a lamp emitting at a UV wavelength. It is also possible to implement the solution so that one UV radiator emits at two or more UV wavelengths. In the examples, however, embodiments will be presented, in which the first UV radiation and the second UV radiation are emitted by separate UV radiators.

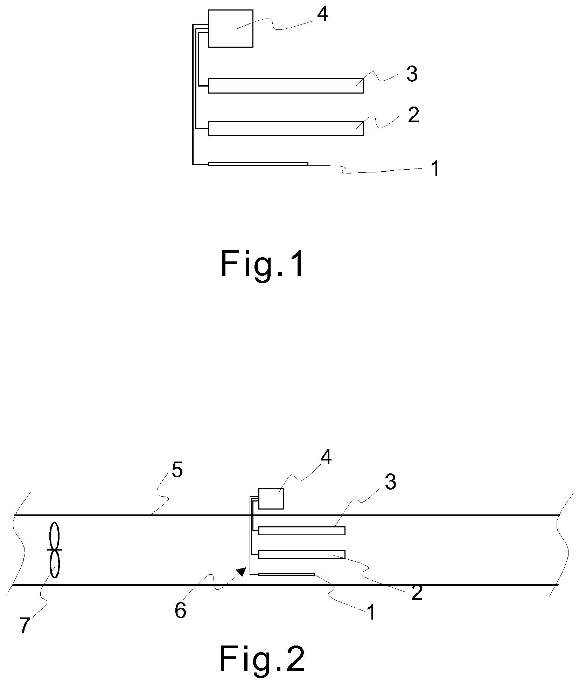

Figure 1 shows those parts in the structure of a dry disinfection device which have a primary effect on the disinfection of air. The figure shows a high-voltage discharge tip used as an ionizer 1, the first UV radiator 2 and the second UV radiator 3. The high-voltage discharge tip 1 (or ionizer) is arranged to generate ions. The first UV radiator 2 is arranged to emit at a first wavelength (wavelength shorter than 200 nm), which radiation advantageously produces ozone. The second UV radiator 3 is arranged to emit at a second wavelength (wavelength longer than 200 nm), which radiation advantageously decontaminates ambient air around the radiator. The ionizer 1 and the UV radiators 2, 3 are used to generate the negative ions, OH radicals and ozone, which are effective in eliminating contaminants, moulds, viruses and bacteria. Furthermore, the figure shows a power supply 4 to supply the ionizer 1 and the UV radiators 2, 3 with the required energy.

Figure 2 shows the placement of the arrangement of Fig. 1 in a ventilation duct 5. A part of the ventilation duct 5 forms a process chamber 6, through which the air to be decontaminated is guided. The movement of the air is generated by a fan 7, which is advantageously a fan of a ventilation system. The negative ions, OH radicals and ozone are generated inside the process chamber 6. For this reason, it is advantageous to use at least two ultraviolet radiation sources 2, 3 emitting at different wavelengths, in addition to the ionizer 1. In the example, the process chamber comprises a high-voltage discharge tip 1 and two ultraviolet radiation sources emitting at different wavelength ranges, *i.e.* the first UV radiator 2 and the second UV radiator 3.

The wavelength of the radiation from the first UV radiator 2 (the first wavelength) is advantageously shorter than 200 nm, preferably 180 to 190 nm, and the wavelength of the radiation from the second UV radiator 3 (the second wavelength) is longer than 200 nm, preferably 245 to 260 nm. In an embodiment, the wavelength of 185 nm is used for ozone production and the wavelength of 253.7 nm *e.g.* for killing bacteria.

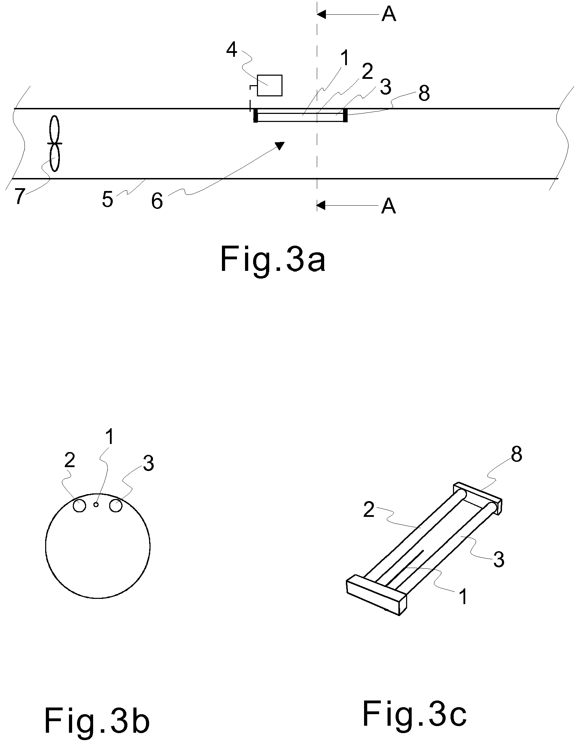

Figures 3a, 3b and 3c show an advantageous embodiment of the dry disinfection device. Figure 3a shows the cross section of the device, seen from the side direction. In the example, the direction of the air flow is from the left to the right. Figure 3b shows the cross-section of the device at line A-A of Fig. 3a. Figure 3c illustrates some main components of the device and their placement in the same frame structure 8. In the example, the device comprises a frame structure 8 to which the first UV radiator 2, the second UV radiator 3 and the ionizer 1 are fixed.

The ionizer 1 can be placed entirely in the process chamber 6, as in the examples above. It is also possible to place the ionizer 1 in such a way that the ions are introduced in the process chamber 6 from the outside of the actual process chamber. For example, the ion generating unit of the ionizer may be placed outside the process chamber 6, and the ions are led from the ion generating unit into the process chamber in a suitable way.

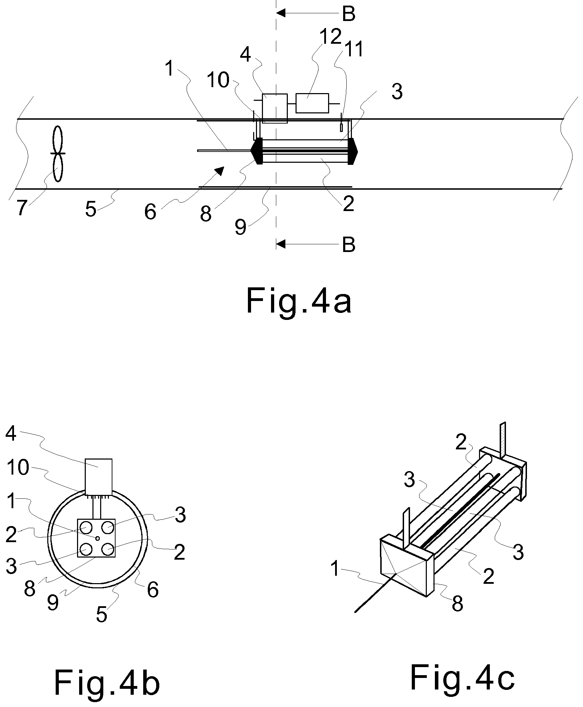

Figures 4a, 4b and 4c show an advantageous embodiment of another dry disinfection device. Figure 4a shows the cross section of the device, seen from the side direction. In the example, the direction of the air flow is also from the left to the right. Figure 4b shows the cross-section of the device at line B-B of Fig. 4a. Figure 4c illustrates some main components of the device and their placement in the same frame structure 8. In the example, the first UV radiator 2, the second UV radiator 3 and the ionizer 1 are fixed to the frame structure 8 which is placed in the centre of the process chamber 6. In this way, air flows substantially all around the frame structure 6. In the example, there are two first UV radiators 2 (in the example, UV lamps) and two UV radiators 3 (in the example, UV lamps). The ionizer 1, in turn, is placed at the end of the frame structure 8 (in the example, at the end from which air flows into the process chamber 6).

In the embodiment of Figs. 4a, 4b and 4c, the process chamber 6 further comprises a reflecting layer 9 that forms the inner wall of the process chamber and is arranged to reflect the radiation of the UV radiators 2, 3 back to the medium flow. In the example, the reflecting layer 9 is also arranged to function as a counter electrode for the discharge tip 1 used as an ionizer.

Preferably, the device comprises a single power supply 4 arranged to supply electricity to the first UV radiator 2, the second UV radiator 3 and the ionizer 1. In the example shown in Figs. 4a and 4b, the cooling ribs 10 of the power supply 4 are placed inside the ventilation duct 5, wherein the cooling of the power supply is efficient and no separate fan is needed in the power supply.

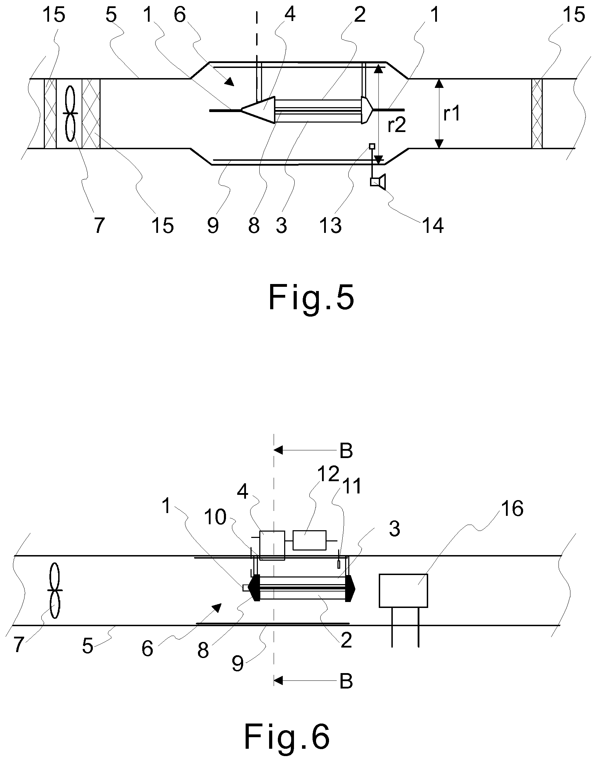

Figure 5, in turn, shows another embodiment, in which the frame structure 8 is placed in the centre of the process chamber 6. In the example, there are two first UV radiators 2 and two second UV radiators 3. There are two ionizers 1, and they are placed at each end of the frame structure 8. In the example, the power supply 4 is also placed in the frame structure 8, wherein it is in an air flow that has an advantageous effect on the cooling of the power supply. The power supply 4 may also be on the wall or outside of the process chamber 6, wherein the required energy is supplied by conductors from the power supply to the frame structure 8. The solution according to the example is advantageous when large quantities of air are involved. In this case, it is possible to use a process chamber 6 with a large diameter, through which even a large quantity of air can be transferred at reasonable flow rates.

In the examples of Figs. 2, 3a, 3b, 3c, 4a, 4b, 4c, and 5, the walls of the process chamber 6 consist of a ventilation duct 5. By the shape of the ventilation duct 5 it is possible to affect the air flow rate and thereby the duration of the process. In the embodiment shown in Fig. 5, the shape of the ventilation duct 5 is expanding at the process. In other words, the diameter r2 of the process chamber 6 is larger than the diameter r1 of the rest of the duct 5. By increasing the diameter r2 of the process chamber 6, the air flow rate in the process chamber is reduced and the disinfection process becomes more efficient.

The dimensions of the device constitute an essential factor on the efficiency of the process. For example, the device and the process chamber 6 can be suitably dimensioned by employing the following dimensioning rules based on the measurements of the device and the duct and the retention time.

L = length of the process chamber

V = rate of the air flowing in the tube size to be connected

A = cross-sectional area in the process chamber per cross-sectional area in the duct, to which the device is coupled

(boundary conditions: $A \geq 1$ and $0 \leq V \leq 10$ m/s)

$$\text{Retention time} = (L*A)/(V)$$

The retention time of the flow entering the process chamber 6 should preferably be 0.4 to 2 seconds. In locations with mould, for example, the optimal retention time is 0.8 seconds or longer. In an advantageous embodiment, UV light is generated at a power of 20 to 250 W and ionized at a power of 4 to 200 W for each 1 $m^3$. The ratio between ionization and UV light can be varied according to the use. Other components can also be varied, depending on the problem.

In an advantageous embodiment, the frame structure 8 is connected to a kind of a cover structure. The cover structure is used at the process chamber 6 as a lid for the ventilation duct 5 and as a mounting support for the device. Thus, by opening the lid, the device can also be taken out of the process chamber 6, for example for maintenance.

In the embodiments shown as examples, the fans 7 of the ventilation duct are advantageously responsible for generating the air flow, so that no separate fans will be needed in the disinfection device.

In an advantageous embodiment, such as the example of Fig. 4a, means 11 are provided for measuring the contents of ions, OH radicals and/or ozone, and/or for measuring the quantity of UV radiation. On the basis of the measurement data, the operation of the device is adjusted to keep the values within the desired limit values. For example, a control unit 12 can be used for the control.

In an advantageous embodiment, such as the example of Fig. 5, the process chamber comprises a UV radiation detector 13, from which the data is transmitted to the outside of the process chamber. In this case, alarm data is received at an alarm device 14 if the UV radiator 2, 3 stops functioning (for example, the UV lamp goes out). Preferably, there are UV radiation detectors 13 for each wavelength to be used, wherein alarm data is obtained on which UV radiator 2, 3 is out of order. On the basis of the alarm data, the damaged UV radiator 2, 3 can also be repaired between periodic inspections. Preferably, the time span between the periodic inspections can be prolonged, and in this way, the total time spent on the periodic inspections is reduced.

In an advantageous embodiment, the operation of the device is controlled by monitoring the energy consumption of the device. If the energy consumption deviates from the limit values for a given time, the device is regarded as having been damaged, and an alarm is given. For example, the going out of the UV lamp 2, 3 reduces the energy consumption of the device, which can be detected by measuring. If the energy consumption remains at a low level for a set time, an alarm is given. By using a defined period of time, during which the energy consumption must be outside the limit values, alarms due to temporary power fluctuations are avoided.

In an advantageous embodiment, such as in the example of Fig. 6, means 16 are provided for removing electrically charged particles from the air flow. For example, the device 16 may be an electric diffusion collector with preferably a sufficient electric field for destroying ozone. Preferably, the voltage of the diffusion collector 16 is optimized according to the desired size of particles to be collected.

Furthermore, the ventilation system may comprise various auxiliary structures 15, as shown in Fig. 5. For example, it is possible to use activated carbon filters, honeycombs and/or coarse filters. A honeycomb 15 is a cell formed by small parallel channels, through which the air flow is guided. The honeycomb 15, which is preferably made of a luminescent material, is placed upstream and/or downstream of the process chamber 6. Thus, the UV light is reflected within the channels of the honeycomb 15, wherein the particles of the flow are subjected more efficiently to UV radiation. By a suitable design of the honeycombs 15, the flow can be made laminar or turbulent. In an advantageous embodiment, the flow is made turbulent when entering the process chamber 6. Preferably, the honeycombs 15 are used to make the flow parallel. In an embodiment shown in Fig. 5, the two auxiliary structures indicated with the reference numeral 15 on the right-hand side are honeycombs, and the structure on the left-hand side is an activated carbon filter. With the activated carbon filter, the residue of the decontamination by negative ions, OH radicals and ozone can be effectively collected.

Furthermore, the ventilation system typically comprises various filters 15, such as coarse filters. Typically, there are filters in the vicinity of air intake, but they may also be provided elsewhere, such as for example in connection with air outlet, as shown in Fig. 5. The present arrangement provides an efficient way to decontaminate an air flow, and this allows the use of more coarse filters. More coarse filters refer to filters which filter larger particles. The pressure loss caused by such filters is typically smaller than that caused by finer filters. Consequently, with the presented dry disinfection arrangement, it is possible to use coarse filters with a smaller pressure loss and thereby to achieve the air flow with less work.

By combining, in various ways, the modes and structures disclosed in connection with the different embodiments of the invention presented above, it is possible to produce various embodiments of the invention in accordance with the spirit of the invention. Therefore, the above-presented examples must not be interpreted as restrictive to the invention, but the embodiments of the invention may be freely varied within the scope of the inventive features presented in the claims hereinbelow.

**Claims**

1.    A dry disinfection device for disinfecting air, the device comprising at least

   - a process chamber (6), wherein the process chamber (6) is part of a ventilation duct (5) and wherein the air to be decontaminated is guided through the process chamber (6) and wherein the process chamber (6) comprises at least a UV radiator (2) arranged to emit at a first wavelength and arranged to produce ozone, the process chamber (6) further comprising a UV radiator (3) arranged to emit at a second wavelength, which second wavelength is different from the first wavelength;
   - an ionizer (1) arranged to generate ions for the process chamber (6),
   - a diffusion collector arranged to remove particles from the air,
   **characterized in that** the process chamber (6) is dimensioned by the following dimensioning rules based on the measurements of the device and the duct (5) and a retention time, wherein the equation is

$$\text{Retention time} = \frac{L * A}{V},$$

wherein,

retention time stands for a time of air flowing in the process chamber (6), and wherein the retention time is 0.4 - 2 seconds,
L stands for the length of the process chamber (6),
A stands for the cross-sectional area in the process chamber (6) per the cross-sectional area in the duct (5), and
V stands for a rate of the air flowing through the duct (5) and wherein A≥1 and $0 \leq V \leq 10$ m/s.

2. The dry disinfection device according to claim 1, **characterized in that** the process chamber (6) further comprises a layer (9) that forms the inner wall of the process chamber (6) and is arranged to reflect UV radiation back to a medium flow.

3. The dry disinfection device according to claim 2, **characterized in that** the ionizer (1) is a high-voltage discharge tip and the layer (9) is also arranged to function as a counter electrode for the discharge tip.

4. The dry disinfection device according to any of the preceding claims, **characterized in that** the device comprises a first UV radiator (2) arranged to emit at a first wavelength, and a second UV radiator (3) arranged to emit at a second wavelength.

5. The dry disinfection device according to claim 4, **characterized in that** the device also comprises a power supply arranged to supply electricity to the first UV radiator (2), the second UV radiator (3) and the ionizer (1).

6. The dry disinfection device according to claim 4 or 5, **characterized in that** the device further comprises a frame structure (8), to which the first UV radiator (2), the second UV radiator (3) and the ionizer (1) are fixed.

7. The dry disinfection device according to any of the preceding claims, **characterized in that** the first wavelength is shorter than 200 nm.

8. The dry disinfection device according to any of the preceding claims, **characterized in that** the first wavelength is longer than 200 nm.

9. The dry disinfection device according to any of the preceding claims, **characterized in that** the walls of the process chamber (6) consist of a ventilation duct (5).

10. A method for decontaminating air, the method comprising

- guiding an air flow of air to be decontaminated through a process chamber (6) of a duct (5), and where the duct (5) comprises at least a dimension for a cross-sectional area,
- producing ozone by first UV radiation into the process chamber (6),
- emitting second UV radiation, the wavelength of the second radiation being longer than the wavelength of the first radiation,
- producing ions with an ionizer (1) into the process chamber (6),
- producing OH radicals into the process chamber (6),
- removing particles from the air flow by a diffusion collector after a treatment with the UV radiation, ozone, ions and OH radicals,
**characterized in that** the process chamber (6) is dimensioned by the following dimensioning rules based on the measurements of the device and the duct (5) and a retention time, wherein the equation is

$$\text{Retention time} = \frac{L * A}{V},$$

wherein,

retention time stands for a time of air flowing in the process chamber (6), and wherein the retention time is 0.4 - 2 seconds,
L stands for the length of the process chamber (6),

A stands for the cross-sectional area in the process chamber (6) per the cross-sectional area in the duct (5), and
V stands for a rate of the air flowing through the duct (5) and wherein A≥1 and $0 \leq V \leq 10$ m/s.

**11.** The method according to claim 10, **characterized in that** the wavelength of the first radiation is shorter than 200 nm and the wavelength of the second radiation is longer than 200 nm.


**Patentansprüche**

**1.** Eine Trocken-Desinfektionsvorrichtung zum Desinfizieren von Luft, wobei die Vorrichtung mindestens Folgendes aufweist:

- eine Prozesskammer (6), wobei die Prozesskammer (6) ein Teil einer Lüftungsleitung (5) ist und wobei die zu dekontaminierende Luft durch die Prozesskammer (6) geleitet wird und wobei die Prozesskammer (6) mindestens einen UV-Strahler (2) umfasst, der angeordnet ist, bei einer ersten Wellenlänge abzustrahlen und angeordnet ist, Ozon zu erzeugen, wobei die Prozesskammer (6) darüber hinaus einen UV-Strahler (3) umfasst, der angeordnet ist, bei einer zweiten Wellenlänge abzustrahlen, wobei sich die zweite Wellenlänge von der ersten Wellenlänge unterscheidet;
- einen Ionisator (1), der angeordnet ist, Ionen für die Prozesskammer (6) zu erzeugen,
- einen Diffusionssammler, der angeordnet ist, Partikel aus der Luft zu entfernen,
**dadurch gekennzeichnet, dass** die Prozesskammer (6) durch die folgenden Bemessungsregeln basierend auf den Maßen der Vorrichtung und der Leitung (5) und einer Rückhaltezeit bemessen ist, wobei die Gleichung folgendermaßen lautet:

$$\text{Rückhaltezeit} = \frac{L * A}{V} \, ,$$

wobei,

Retentionszeit für eine Zeit der Luftströmung in der Prozesskammer (6) steht, und wobei die Rückhaltezeit 0,4 - 2 Sekunden beträgt,
L für die Länge der Prozesskammer (6) steht,
A für die Querschnittsfläche in der Prozesskammer (6) pro Querschnittsfläche im Kanal (5) steht, und
V für eine Geschwindigkeit der durch die Leitung (5) strömenden Luft steht und
wobei A≥1 und $0 \leq V \leq 10$ m/s.

**2.** Trocken-Desinfektionsvorrichtung nach Anspruch 1, **dadurch gekennzeichnet, dass** die Prozesskammer (6) darüber hinaus eine Schicht (9) aufweist, die die Innenwand der Prozesskammer (6) bildet und angeordnet ist, die UV-Strahlung an einen Mediumstrom zurück zu reflektieren.

**3.** Trocken-Desinfektionsvorrichtung nach Anspruch 2, **dadurch gekennzeichnet, dass** der Ionisator (1) eine Hochspannungs-Entladungsspitze ist und die Schicht (9) auch angeordnet ist, als eine Gegenelektrode für die Entladungsspitze zu fungieren.

**4.** Trocken-Desinfektionsvorrichtung nach einem der vorhergehenden Ansprüche, **dadurch gekennzeichnet, dass** die Vorrichtung einen ersten UV-Strahler (2) umfasst, der angeordnet ist, bei einer ersten Wellenlänge abzustrahlen, und einen zweiten UV-Strahler (3), der angeordnet ist, bei einer zweiten Wellenlänge abzustrahlen.

**5.** Trocken-Desinfektionsvorrichtung nach Anspruch 4, **dadurch gekennzeichnet, dass** die Vorrichtung auch eine Stromquelle aufweist, die zum Zuführen von Elektrizität zum ersten UV-Strahler (2), zum zweiten UV-Strahler (3) und zum Ionisator (1) angeordnet ist.

**6.** Trocken-Desinfektionsvorrichtung nach Anspruch 4 oder 5, **dadurch gekennzeichnet, dass** die Vorrichtung darüber hinaus eine Rahmenstruktur (8) umfasst, an der der erste UV-Strahler (2), der zweite UV-Strahler (3) und der Ionisator (1) befestigt sind.

**7.** Trocken-Desinfektionsvorrichtung nach einem der vorhergehenden Ansprüche, **dadurch gekennzeichnet, dass** die erste Wellenlänge kürzer als 200 nm ist.

**8.** Trocken-Desinfektionsvorrichtung nach einem der vorhergehenden Ansprüche, **dadurch gekennzeichnet, dass** die erste Wellenlänge länger als 200 nm ist.

**9.** Trocken-Desinfektionsvorrichtung nach einem der vorhergehenden Ansprüche, **dadurch gekennzeichnet, dass** die Wände der Prozesskammer (6) aus einem Lüftungsleitung (5) bestehen.

**10.** Ein Verfahren zum Dekontaminieren von Luft, wobei das Verfahren Folgendes umfasst:

- Leiten eines Luftstroms von zu dekontaminierender Luft durch eine Prozesskammer (6) einer Leitung (5), wobei die Leitung (5) mindestens eine Abmessung für eine Querschnittfläche umfasst,
- Erzeugen von Ozon durch die erste UV-Strahlung in der Prozesskammer (6),
- Ausstrahlen der zweiten UV-Strahlung, wobei die Wellenlänge der zweiten Strahlung länger als die Wellenlänge der ersten Strahlung ist,
- Erzeugen von Ionen mit einem Ionisator (1) in der Prozesskammer (6),
- Erzeugen von OH-Radikalen in der Prozesskammer (6),
- Entfernen von Partikeln aus dem Luftstrom durch einen Diffusionssammler nach einer Behandlung mit UV-Strahlung, Ozon, Ionen und OH-Radikalen,
**dadurch gekennzeichnet, dass** die Prozesskammer (6) durch die folgenden Bemessungsregeln basierend auf den Maßen der Vorrichtung und der Leitung (5) und einer Rückhaltezeit bemessen ist, wobei die Gleichung folgendermaßen lautet:

$$\text{Rückhaltezeit} = \frac{L * A}{V},$$

wobei,

Retentionszeit für eine Zeit der Luftströmung in der Prozesskammer (6) steht, und wobei die Rückhaltezeit 0,4 - 2 Sekunden beträgt,
L für die Länge der Prozesskammer (6) steht,
A für die Querschnittfläche in der Prozesskammer (6) pro Querschnittfläche in der Leitung (5) steht, und
V für einen Geschwindigkeit der durch den Kanal (5) strömenden Luft steht und wobei $A \geq 1$ und $0 \leq V \leq 10$ m/s.

**11.** Verfahren nach Anspruch 10, **dadurch gekennzeichnet, dass** die Wellenlänge der ersten Strahlung kürzer als 200 nm und die Wellenlänge der zweiten Strahlung länger als 200 nm ist.

**Revendications**

**1.** Dispositif de désinfection par voie sèche pour désinfecter l'air, le dispositif comprenant au moins

- une chambre de traitement (6), dans lequel la chambre de traitement (6) fait partie d'une conduite de ventilation (5) et dans lequel l'air devant être décontaminé est guidé à travers la chambre de traitement (6) et dans lequel la chambre de traitement (6) comprend au moins un radiateur UV (2) agencé pour émettre à une première longueur d'onde et agencé pour produire de l'ozone, la chambre de traitement (6) comprenant en outre un radiateur UV (3) agencé pour émettre à une deuxième longueur d'onde, laquelle deuxième longueur d'onde est différente de la première longueur d'onde ;
- un ioniseur (1) agencé pour générer des ions pour la chambre de traitement (6),
- un collecteur de diffusion agencé pour éliminer des particules de l'air,
**caractérisé en ce que** la chambre de traitement (6) est dimensionnée par les règles de dimensionnement suivantes sur la base des mesures du dispositif et de la conduite (5) et d'une durée de rétention, dans lequel l'équation est

$$\text{Durée de rétention} = \frac{L*A}{V},$$

dans laquelle,

durée de rétention représente une durée d'écoulement d'air dans la chambre de traitement (6), et dans laquelle la durée de rétention est 0,4-2 seconde(s),
L représente la longueur de la chambre de traitement (6),
A représente la superficie en coupe transversale dans la chambre de traitement (6) par la superficie en coupe transversale dans la conduite (5), et
V représente un débit de l'air traversant la conduite (5) et dans laquelle $A \geq 1$ et $0 \leq V \leq 10$ m/s.

**2.** Dispositif de désinfection par voie sèche selon la revendication 1, **caractérisé en ce que** la chambre de traitement (6) comprend en outre une couche (9) qui forme la paroi intérieure de la chambre de traitement (6) et est agencée pour réfléchir un rayonnement UV en retour jusqu'à un flux de milieu.

**3.** Dispositif de désinfection par voie sèche selon la revendication 2, **caractérisé en ce que** l'ioniseur (1) est une extrémité de décharge haute tension et la couche (9) est également agencée pour fonctionner comme une contre-électrode pour l'extrémité de décharge.

**4.** Dispositif de désinfection par voie sèche selon l'une quelconque des revendications précédentes, **caractérisé en ce que** le dispositif comprend un premier radiateur UV (2) agencé pour émettre à une première longueur d'onde, et un deuxième radiateur UV (3) agencé pour émettre à une deuxième longueur d'onde.

**5.** Dispositif de désinfection par voie sèche selon la revendication 4, **caractérisé en ce que** le dispositif comprend également une alimentation électrique agencée pour fournir de l'électricité au premier radiateur UV (2), au deuxième radiateur UV (3) et à l'ioniseur (1).

**6.** Dispositif de désinfection par voie sèche selon la revendication 4 ou 5, **caractérisé en ce que** le dispositif comprend en outre une structure de châssis (8), à laquelle le premier radiateur UV (2), le deuxième radiateur UV (3) et l'ioniseur (1) sont fixés.

**7.** Dispositif de désinfection par voie sèche selon l'une quelconque des revendications précédentes, **caractérisé en ce que** la première longueur d'onde est plus courte que 200 nm.

**8.** Dispositif de désinfection par voie sèche selon l'une quelconque des revendications précédentes, **caractérisé en ce que** la première longueur d'onde est plus longue que 200 nm.

**9.** Dispositif de désinfection par voie sèche selon l'une quelconque des revendications précédentes, **caractérisé en ce que** les parois de la chambre de traitement (6) consistent en une conduite de ventilation (5).

**10.** Procédé de décontamination d'air, le procédé comprenant

- le guidage d'un flux d'air devant être décontaminé à travers une chambre de traitement (6) d'une conduite (5), et où la conduite (5) comprend au moins une dimension pour une superficie en coupe transversale,
- la production d'ozone par un premier rayonnement UV dans la chambre de traitement (6),
- l'émission d'un deuxième rayonnement UV, la longueur d'onde du deuxième rayonnement étant plus longue que la longueur d'onde du premier rayonnement,
- la production d'ions avec un ioniseur (1) dans la chambre de traitement (6),
- la production de radicaux OH dans la chambre de traitement (6),
- l'élimination de particules du flux d'air par un collecteur de diffusion après un traitement avec le rayonnement UV, l'ozone, les ions et les radicaux OH,
**caractérisé en ce que** la chambre de traitement (6) est dimensionnée par les règles de dimensionnement suivantes sur la base des mesures du dispositif et de la conduite (5) et d'une durée de rétention, dans lequel l'équation est

$$\text{Durée de rétention} = \frac{L*A}{V},$$

dans laquelle,

durée de rétention représente une durée d'écoulement d'air dans la chambre de traitement (6), et dans laquelle la durée de rétention est 0,4-2 seconde(s),
L représente la longueur de la chambre de traitement (6),
A représente la superficie en coupe transversale dans la chambre de traitement (6) par la superficie en coupe transversale dans la conduite (5), et
V représente un débit de l'air traversant la conduite (5) et dans laquelle A $\geq$ 1 et 0 $\leq$ V $\leq$ 10 m/s.

**11.** Procédé selon la revendication 10, **caractérisé en ce que** la longueur d'onde du premier rayonnement est plus courte que 200 nm et la longueur d'onde du deuxième rayonnement est plus longue que 200 nm.

Fig.1

Fig.2

Fig.3a

Fig.3b

Fig.3c

# Fig.4a

# Fig.4b

# Fig.4c

Fig.5

Fig.6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070041882 A